# EUROPEAN PATENT SPECIFICATION

(11) **EP 1 377 276 B1**
(45) Date of publication and mention of the grant of the patent: **05.10.2011**
(21) Application number: 02733207.1
(22) Date of filing: 09.04.2002
(51) Int. Cl.: A61K 9/24, A61K 9/36, A61K 47/38

(54) **TIMED PULSE RELEASE COMPOSITION**
ZUBEREITUNG ZUR GETAKTETEN FREISETZUNG
COMPOSITION A LIBERATION PAR IMPULSION REGLEE DANS LE TEMPS

(30) Priority: 10.04.2001 IN MU03252001
(43) Date of publication of application: 07.01.2004
(73) Proprietor: Sun Pharma Advanced Research Company Limited, Mumbai 400 093 (IN)
(72) Inventor: SHANGHVI, Dilip Shantilal, Andheri (East), 400059 Mumbai (IN); DHARMADHIKARI, Nitin Bhalachandra, 400098 Mumbai (IN); ZALA, Yashoraj Rupsinh, 400098 Mumbai (IN); KHANNA, Satish C., 4103 Basle (CH)
(74) Representative: Watson, Robert James
(86) International application number: PCT/IN2002/000107
(87) International publication number: WO 2002/080887

(56) References cited:
- EP-A1- 0 396 404
- US-A- 5 188 841
- US-A- 5 882 656
- US-A- 6 096 341
- PATENT ABSTRACTS OF JAPAN & JP 60 241 871 A (AJINOMOTO KK) 30 November 1985

## Description

### FIELD OF THE INVENTION

The present invention relates to a timed pulse release composition, which releases in a pulse, the therapeutically active agent in a reliable manner at about a predetermined time.

### BACKGROUND OF THE INVENTION

A plethora of prior arts relate to pharmaceutical compositions that release a drug after a delay. Some prior arts that relate to release of drug after a predetermined time include United States Patent No 3,247,066; European Patent No. EP 0 408 496; United States Patent No. 4,871,549; United States Patent No. 5,229,131; PCT Publication No. WO 9918938 and PCT Publication No. WO 074655. All of these relate to systems comprising a core that swells upon imbibing fluid from the surrounding and a coat that ruptures due to the pressure exerted upon it by the swelling core. Prior arts such as United States Patent No. 3,247,066, European Patent EP 1123700, United States Patent No. 5,260,069, and United States Patent No. 4,871,549 are distinct from the present invention in that they relate to controlled release dosage forms. Herein the dose of the drug is divided in multiple units and there is no specific and particular requirement of assurance that a unit ruptures at a predetermined time in a reliable manner. Statistically, different units rupture at different times and thereby provide controlled release of active ingredient, on an average, over a period of time. In the present invention, the total amount of active ingredient is contained in one single unit and is intended to be released as a pulse at the predetermined time. An important requirement for using such systems in large number of patients is that the system should deliver the drug as a pulse at about the predetermined time in a reliable manner to the large number of patients to whom the system is administered. Thus, the coat rupture should occur reliably, the core should disintegrate immediately and consequently the drug should be released as a pulse reliably. For instance, if in five to ten out of a hundred times the coatings do not open or rupture at about the predetermined time but rupture at a significantly prolonged time when tested by agitation over a range of agitational conditions and aqueous compositions, then the desired release at the predetermined time is not achieved reliably. Also, if the release prior to rupture or the rupture time is significantly influenced by changes in pH, composition of the surrounding fluids and the agitation conditions, then the desired release at the predetermined time is not achieved reliably. Also, if the coat rupture occurs but the therapeutically active agent is not released as a pulse in all or some of the units, then the desired release as a pulse at a predetermined time is not achieved reliably. Prior arts such as WO 99/18938, WO 074655, and EP 0 408 496 make no reference to reliability of rupture or release from a large number of tablets, or to optimizing the compositions to obtain the reliability of rupture or reliability of release over a large number of tablets. United States Patent No. 5,229,131 presents a large amount of data giving the percent tablets splitting at 30 min and 60 min and the percent tablets releasing their contents at 60 min and 120 min in Tables 12 to 18. The tablets do not provide reliable manner of rupture as provided by the composition of the present invention, wherein 36 out of 36 tablets rupture within ± 50% of the coating rupture time. Despite the plethora of prior art, there are no commercially successful systems of a timed pulse release composition comprising:
a. a core composition comprising a therapeutically active agent, a swelling agent, and optionally water soluble compound(s) for inducing osmosis, and
b. a coat composition comprising one or more film forming polymers,
   wherein upon imbibing fluid from the surrounding the core swells, and the coat ruptures to release in a pulse, the therapeutically active agent in a reliable manner, at about a predetermined time after oral administration of the composition. Further, there is no prior art that discloses such compositions with reliability of rupture in an in-vivo situation wherein the tablets are administered to human subjects. The timed pulse release composition of the present invention has these desirable attributes such that the coat ruptures and releases as a pulse the therapeutically active agent in a reliable manner at about a predetermined time after oral administration of the composition.

### OBJECT OF THE INVENTION

It is an object of the present invention to provide a timed pulse release composition comprising a swellable core and a coat wherein upon imbibing fluid from the surrounding the core swells, and the coat ruptures to release in a pulse, the therapeutically active agent in a reliable manner at about a predetermined time.

It is a further object to provide a timed pulse release composition that performs reliably in human patients. Accordingly, it is an object of the present invention to provide a timed pulse release composition wherein upon oral administration of the composition to human subjects, the coat ruptures in a reliable manner at about a predetermined time after oral administration of the composition.

### SUMMARY OF THE INVENTION

The present invention provides a timed pulse release composition according to claim 1.

### DETAILED DESCRIPTION OF THE INVENTION

The present invention provides a timed pulse release composition comprising:
a. a core composition comprising a therapeutically active agent, a swelling agent, and optionally water soluble compound(s) for inducing osmosis, and
b. a coat composition comprising one or more film forming polymers,
   wherein upon imbibing fluid from the surrounding the core swells, and the coat ruptures to release in a pulse, the therapeutically active agent in a reliable manner at about a predetermined time wherein the reliable manner of rupture comprises rupturing of 36 tablets out of a total of 36 tablets at about the predetermined time when tested by subjecting the tablets to USP dissolution test using an aqueous media at 37±0.5°C, in a USP Type I or Type II apparatus at an rpm selected from the range of about 50 rpm to about 100 rpm. Further wherein the predetermined time is in the range of about 1 hour to about 4 hours, the 36 out of the 36 tablets rupture within about ± 50% of the predetermined time; and wherein the predetermined time is in the range of about >4 hours to about 12 hours, the 36 out of the 36 tablets rupture within about ± 25% of the predetermined time.

According to the present invention, the timed pulse release composition imbibes fluids from the environment of use causing the swelling agent in the core to swell. The therapeutically active agent is then released as a pulse after the timed pulse release coat ruptures under the influence of mechanical pressure exerted by the swelling of the swelling agent(s) present in the core. The time of rupture of the coat can be controlled by varying (a) the degree and rate of swelling of the core; (b) the timed pulse release coat composition, by using different components and ratios of these components; and (c) the thickness of the coat.

The therapeutically active agent may be selected from the therapeutic class viz. alcohol anti-abuse preparations, drugs used for Alzheimer's disease, anesthetics, acromegaly agents, analgesics, antiasthmatics, anticancer agents, anticoagulants, antithrombotic agents, anticonvulsants, antidiabetics, antiemetics, antiglaucoma agents, antihistamines, anti-infective agents, antiparkinsons agents, antiplatelet agents, antirheumatic agents, antispasmodics, anticholinergic agents, antitussives, carbonic anhydrase inhibitors, cardiovascular agents, cholinesterase inhibitors, agents for the treatment of CNS disorders, CNS stimulants, cystic fibrosis management agents, dopamine receptor agonists, agents for endometriosis management, erectile dysfunction therapy, fertility agents, gastrointestinal agents, immunomodulators, immunosuppressives, memory enhancers, migraine preparations, muscle relaxants, nucleoside analogues, osteoporosis management agents, parasympathomimetics, prostaglandins, psychotherapeutic agents, sedatives, hypnotics, tranquillizers, drugs used for skin ailments, steroids and hormones.

The term "release as a pulse" refers to release characteristic of conventional tablets and capsules that are devoid of design characteristics that result in slow, extended, controlled or retarded release of the therapeutically active agent. For example, in a particular embodiment where the predetermined time of pulse release is about 70 min, the "release of therapeutically active agent as a pulse" comprises release of not more than 10% of the active ingredient at 45 min and at least 70% of the active ingredient at 2 hrs, when tested by subjecting the tablets to USP dissolution test using pH 6.8 buffer at 37±0.5°C, in a USP Type II apparatus at an rpm of 75.

The swelling agent used in the timed pulse release composition includes one or more swellable hydrophilic polymers. The quantity or relative proportion of the polymers is subject to considerable variation. However, a sufficient quantity of the material is present in the core to provide, upon uptake of water, a swelling pressure in excess of the cohesive strength of the coating surrounding the tablet or core. Preferably, the polymers are employed in the dry state or in a form that has substantial capacity for water uptake. Examples of swellable hydrophilic polymers that may be used in the timed pulse release composition of the present invention as the swelling agent include vinylpyrrolidone polymers such as povidone, or crosslinked polyvinylpyrrolidone such as crospovidone; cellulose and cellulose derivatives such as microcrystalline cellulose, methylcellulose, ethylcellulose, hydroxypropylcellulose, hydroxypropyl methylcellulose, carboxyalkyl celluloses or crosslinked carboxyalkylcelluloses and their alkali salts; sodium starch glycolate, starch and starch derivatives, ion-exchange resins and mixtures thereof. Preferably, the swelling agent used comprises a swelling agent that swells considerably but does not form a strong gel, and may be selected from the group comprising crosslinked sodium carboxymethyl cellulose, crosslinked polyvinylpyrrolidone and sodium starch glycolate.

The alkali salt of crosslinked carboxyalkyl cellulose, i.e. crosslinked sodium carboxymethyl cellulose, also known as croscarmellose sodium or Ac-Di-Sol, is available commercially as Nymcel^{®} ZSX, Pharmacel^{®} XL, Primellose^{®} or Solutab^{®}. The amount of swelling agent that may be used is dependent on the desired time of rupture of the timed pulse release coat, nature and amounts of other components in the core, as well as the composition and thickness of the coat. Generally, croscarmellose sodium may be used as the polymeric swelling agent in an amount ranging from about 0.5% to about 50% by weight of the core, preferably from about 2% to about 40% by weight of the core, more preferably from about 5% to about 20% by weight of the core. In specific preferred embodiments, croscarmellose sodium is used in a range from about 6% to about 10% by weight of the core, more preferably from about 7% to about 9% by weight of the core.

Vinyl pyrrolidone polymers or polyvinyl pyrrolidone (PVP), also referred to as Povidone, are synthetic polymers consisting essentially of linear 1-vinyl-2-pyrrolidinone groups, the degree of polymerization of which results in polymers of various molecular weights, the molecular weight ranging between 2500 and 3,000,000 Daltons. PVP is commercially available as Kollidon^{®} (BASF), Plasdone^{®} and Peristone^{®} (General Aniline). PVP is classified into different grades on the basis of its viscosity in aqueous solution. Different grades of PVP available are PVP K-12, PVP K-15, PVP K-17, PVP K-25, PVP K-30, PVP K-60, PVP K-90 and PVP K-120. The K-value referred to in the above nomenclature is calculated from the viscosity of the PVP in aqueous solution, relative to that of water. Crospovidone or cross-PVP, the synthetic crosslinked homopolymer of N-vinyl-2-pyrrolidinone, may also be used as a swellable hydrophilic polymer.

It is commercially available as Kollidon CL and Polyplasdone XL, and has a molecular weight higher than 1,000,000 Daltons. Crospovidone is used in the present invention in an amount ranging from about 2% to about 5% by weight of the core. The preferred vinyl pyrrolidone polymer for use as a swellable hydrophilic polymer is PVP K-30, having an approximate molecular weight of 50,000 Daltons. It may be used in an amount ranging from about 0.5% to about 5% by weight of the core, more preferably from about 1% to about 2% by weight of the core.

Sodium starch glycolate, the sodium salt of carboxymethyl ether of starch, may also be used as the polymeric swelling agent. It has a molecular weight ranging between 500,000 and 1,000,000 Daltons, and is commercially available as Explotab and Primojel. Sodium starch glycolate may be used in the present invention in an amount ranging from about 0.5% to about 40% by weight of the core, preferably from about 2% to about 40% by weight of the core, more preferably from about 2% to about 10% by weight of the core.

Preferably, the timed pulse release composition of the present invention contains a wicking agent. The term wicking agent as used herein implies a broader definition than a conventional wicking agent and includes any pharmaceutical excipient that provides influx of water into the core by any suitable mechanism, preferably by capillary action as is typical of conventional wicking agents. Materials suitable for use as wicking agents in the timed pulse release composition include, but are not limited to, colloidal silicon dioxide, kaolin, titanium dioxide, fumed silicon dioxide, alumina, sodium lauryl sulfate, microcrystalline cellulose, low molecular weight polyvinyl pyrrolidone, bentonite, magnesium aluminum silicate, and the like. The timed pulse release composition of the present invention may be optimized to obtain the reliable manner of rupture without the use of a wicking agent. However, the use of a wicking agent has been found to be useful in that the task of optimization to obtain the reliable manner of rupture is made easier.

Microcrystalline cellulose (MCC) is used in the preferred embodiment as the wicking agent. It is made up of a chain of about 250 glucose molecules in the form of a microcrystal, consisting primarily of crystallite aggregates obtained by removing amorphous regions of a pure cellulose source material by hydrolytic degradation using mineral acid. MCC has an average molecular weight of about 36,000 Daltons and is available in various grades, which differ in bulk density, particle size and moisture content. It is commercially available as Vivapur^{®}, Avicel^{®}, Vivacel^{®}. Emcocel^{®}, Fibrocel^{®} and Tabulose^{®}. Avicel^{®} PH 102, having a mean particle size of 100µ m, i.e. 8% or less of the particles are retained on a # 60 sieve (as defined by ASTM, American Society for Testing and Materials), and 45% or more of the particles are retained on a #200 sieve (as defined by ASTM), and having a moisture content ≤5%, is used in more preferred embodiments of the timed pulse release composition, in an amount ranging from about 2% to about 5% by weight of the core, more preferably from about 2% to about 3% by weight of the core.

Water-soluble compounds suitable for inducing osmosis, i.e. osmotic agents or osmogents are generally used in the core of the timed pulse release composition when the drug itself does not exert sufficient osmotic pressure in order to imbibe fluid from the surroundings. Osmogents that may be present in the core of the timed pulse release composition include all pharmaceutically acceptable and pharmacologically inert water-soluble compounds referred to in the pharmacopoeias such as United States Pharmacopoeia, as well as in Remington: The Science and Practice of Pharmacy, edition 20, Lippincott Williams and Wilkins, Philadelphia (2000). Pharmaceutically acceptable water-soluble salts of inorganic or organic acids, or non-ionic organic compounds with high water solubility, e.g. carbohydrates such as sugar, or amino acids, are generally preferred. The examples of agents used for inducing osmosis include inorganic salts such as magnesium chloride or magnesium sulfate, lithium, sodium or potassium chloride, lithium, sodium or potassium hydrogen phosphate, lithium, sodium or potassium dihydrogen phosphate, salts of organic acids such as sodium or potassium acetate, magnesium succinate, sodium benzoate, sodium citrate or sodium ascorbate; carbohydrates such as mannitol, sorbitol, arabinose, ribose, xylose, glucose, fructose, mannose, galactose, sucrose, maltose, lactose, raffinose; water-soluble amino acids such as glycine, leucine, alanine, or methionine; urea and the like, and mixtures thereof. The amount of osmogents that may be used depends on the particular osmogent that is used and may range from about 1% to about 60% by weight of the core.

In addition to the above ingredients, the core of the timed pulse release composition may optionally contain pharmaceutically acceptable excipients such as binders, disintegrants, lubricants and the like. Examples of binders used commonly include starch, gelatin, sugars like sucrose, glucose, dextrose, molasses and lactose; acacia, sodium alginate, cellulose derivatives like methyl cellulose, ethyl cellulose, carboxymethyl cellulose and the like; polymers such as polyvinyl pyrrolidone, Veegum, polyethylene glycol, waxes and the like. Examples of lubricants that may be used in the timed pulse release composition include talc, magnesium stearate, calcium stearate, aluminium stearate, stearic acid, hydrogenated vegetable oils, colloidal silicon dioxide, polyethylene glycol, cellulose derivatives such as carboxyalkyl cellulose and its alkali salts, or mixtures thereof. Hydrophobic or water insoluble lubricants may reduce the water imbibing properties of the core whereas hydrophilic or water soluble lubricants do not, and are preferred. A more preferred lubricant is colloidal silicon dioxide. A mixture of colloidal silicon dioxide and magnesium stearate may be used as the preferred lubricant. More preferred embodiments use a combination of microcrystalline cellulose and colloidal silicon dioxide as the wicking agents, with colloidal silicon dioxide also functioning as a lubricant. Colloidal silicon dioxide is available commercially as Aerosil^{®} from Degussa-Huls, Nippon and Fischer GmbH. The preferred colloidal silicon dioxide lubricant is Aerosil^{®} 200, with an approximate external surface area of 200m²/g. The colloidal silica may be used in amounts in the range of about 0.5 % to about 5% by weight of the core.

In a preferred embodiment of the process of making the timed pulse release composition, the core is obtained by mixing the therapeutically active agent and the swelling agent with the binder in a rapid mixer granulator and granulating the mixture. In more preferred embodiments of the present invention, only a part of the total swelling agent is included in the composition and the remaining is mixed at the lubrication stage with the dried granules. The granules obtained using a suitable granulating solvent are wet milled through a screen and then dried in a fluidised bed drier at 40-50°C to a moisture content of 2-3%. The dried granules are then milled through a 2mm screen and are mixed with one or more lubricants and the wicking agent. In more preferred embodiments, as described above, the remaining part of the swelling agent is mixed at this stage. The lubricated granules may be filled in hard gelatin capsules, or may be compressed to obtain the compressed tablets or cores.

The therapeutically active agent comprising compressed cores/capsules are covered with a coat composition comprising one or more film forming polymers, to provide a timed pulse release composition. The film forming polymers that may be used to form this timed pulse release composition are selected from the group consisting of water insoluble polymers, pH dependent polymer, a mixture of water soluble and water insoluble polymers, and mixtures thereof. Examples of film forming polymers that may be used include cellulose ester derivatives like methyl cellulose, ethyl cellulose, hydroxypropyl cellulose, hydroxypropyl methylcellulose, cellulose acetate, cellulose acetate phthalate, pH-independent copolymers of methacrylic acid and methacrylic acid esters commercially available as Eudragit^{®}, or mixtures thereof. The time of release of the therapeutically active agent of the first composition may be varied by varying the components used to form the coat, and/or by varying the ratio in which these components are used. By selecting the suitable components and by using them in suitable ratios, the release can be obtained at about a predetermined time after oral administration of the dosage form. A preferred embodiment of the invention uses a mixture of a water-insoluble polymer and a water soluble polymer to form the delayed release coat. In preferred embodiments ethyl cellulose is used as the water-insoluble polymer and hydroxypropyl methylcellulose (HPMC) is used as the water soluble polymer. The mixture is used in a preferred weight ratio of 0:20 to 20:0 of ethyl cellulose : HPMC, more preferably 6:3 to 9:3.

The coating agents are dispersed in a solvent or solvent system, and the solution or dispersion so obtained is used to coat the cores containing the therapeutically active agent to form the timed pulse release composition. Various solvents and mixtures of solvents can be used to provide the coating agent solution or dispersion. Some of the preferred solvents include water, halogen hydrocarbons like trichloroethylene, methylene chloride (dichloromethane), carbon tetrachloride, and chloroform; alcohols such as absolute alcohol, isopropanol and methanol; low molecular weight esters like ethyl acetate and amyl acetate; and ketones such as acetone, 2-butanone and the like. A preferred embodiment of the present invention uses a mixture of dichloromethane and methanol in a preferred ratio of 1:10 to 10:1 of dichloromethane : methanol, more preferably in a ratio of about 3:1 to about 6:1.

The compressed cores containing the therapeutically active agent are coated with the coating solution to a defined weight gain, the thickness of the coat depending on the predetermined time of release of the active agent. The coating material may be applied by any procedure, which provides a continuous film of essentially uniform thickness. One method of coating involves rotating a bed of uncoated cores in a conventional tablet coating pan and applying a solution or dispersion of the coating agent in a suitable solvent by pouring or spraying the solution onto the moving cores. Other coating procedures such as fluid bed coating, vertical spray coating, and the like can also be employed. The coated cores are dried by exposing them to warm, dry air and may be cured, if necessary, by air drying, baking or force drying. In one embodiment of the present invention, the compressed core is coated with a ethyl cellulose : HPMC solution to a weight gain in the range of about 2% to about 20% by weight of the compressed core. The cores are coated in an automated perforated coating pan followed by drying and curing of the coated cores in a tray drier for 24 hours at 40-50°C.

The following examples do not limit the scope of the invention and are presented as illustrations.

### Example 1

The timed pulse release composition of the present invention was prepared as per the formula in Table 1 below.

**Table 1**

| **Ingredients** | **Quantity (mg)** | **Percent (%) w/w.** |
|---|---|---|
| Metformin hydrochloride | 500.0 | 83.33 |
| Croscarmellose sodium (Ac-Di-Sol) | 50.0 | 8.33 |
| Corn starch, plain (as 10% starch paste) | 17.0 | 2.83 |
| Microcrystalline cellulose (MCC) | 13.5 | 2.25 |
| Colloidal silicon dioxide | 13.5 | 2.25 |
| Magnesium stearate | 6.0 | 1.0 |
| **Total** | **600.0** | **100.0** |
| **Coat**- | | |
| Ethyl cellulose | 40.7 | coated to a weight gain of 9.5% by weight of the core. |
| Hydroxypropyl methylcellulose | 16.3 | |

The method of preparation of the timed pulse release core included sifting the metformin hydrochloride and croscarmellose sodium through a suitable sieve and mixing them in a rapid mixer granulator. The dry powder blend was then granulated using 10% starch paste, followed by wet milling the mass through a Fitz mill. The granules so obtained were dried to a moisture content of 3-4%. The dry granules were then milled in a Fitz mill through a 1.5mm screen, followed by sifting of the granules through a # 16 sieve (as defined by American Society for Testing and Materials, ASTM). These granules of metformin hydrochloride were then mixed with MCC, colloidal silicon dioxide and magnesium stearate, and the lubricated mixture thus obtained was compressed on a rotary compression machine using oblong shaped punches. The tablets were then coated in a conventional coating pan using a solution of ethyl cellulose and HPMC in a mixture of methanol and dichloromethane.

The tablets were subjected to dissolution studies using pH 6.8 buffer at 37±0.5°C, in a USP Type II apparatus (rpm = 75). The release profile for metformin is recorded in Table 2 below. The rupture time of the timed pulse release coating on the core was observed for 30 tablets, which were subjected to dissolution testing. It was found that all the tablets opened reliably at about 1 hour to about 1.3 hour after start of the dissolution test.

**Table 2**

| **Time (mins)** | **% metformin released (±S.D.)** |
|---|---|
| 45 | 1 ± 0.5 |
| 105 | 91 ± 6.89 |
| 120 | 98 ± 4.26 |

The tablets were found to release the metformin as a pulse after the rupture of the coat at a predetermined time.

The tablets were tested in different media, using different conditions of pH and apparatus, and the opening time was recorded. The observations are recorded in Table 3 below.

**Table 3**

| **No.** | **Medium used** | **Conditions used** | **Opening time of 6 tablets (hours.min)** |
|---|---|---|---|
| 1. | pH 6.8 | USP Type I dissolution apparatus, with rpm of 100 | 1.08, 1.25, 1.13, 1.16, 1.02, 1.12 |
| 2. | pH 6.8 | USP Type I dissolution apparatus, with rpm of 100 | 1.04, 1.14, 1.18, 1.09, 1.09, 1.25 |
| 3. | pH 6.8 | USP Type I dissolution apparatus, with rpm of 100 | 1.23, 1.05, 0.59, 1.12, 0.58, 1.25 |
| 4. | pH 6.8 | USP Type I dissolution apparatus, with rpm of 100 | 1.18, 1.26, 1.24, 1.01, 1.12, 1.06 |
| 5. | pH 6.8 | USP Type II dissolution apparatus, with rpm of 75 | 1.28, 1.30, 1.21, 1.17, 1.09, 1.03 |
| 6. | 0.1N HCl | USP Type II dissolution apparatus, with rpm of 75 | 1.07, 1.18, 1.21, 1.10, 1.03, 1.30 |
| 7. | pH 6.8 | USP Type II dissolution apparatus, with rpm of 50 | 1.02, 1.39, 1.28, 1.21, 1.03, 1.26 |
| 8. | 0.1N HCl | USP Type II dissolution apparatus, with rpm of 50 | 1.24, 1.10, 1.05, 1.12, 1.29, 0.50 |

It was found that for 48 out of the 48 tablets that were tested, the coat ruptured within ± 50% of the predetermined time of 70 min. Thus the coat ruptured in a reliable manner.

### Example 2

The timed pulse release composition of the present invention was prepared as per the formula in Table 4 below.

**Table 4**

| **Ingredients** | **Quantity (mg)** | **Percent (%) w/w.** |
|---|---|---|
| Metformin hydrochloride | 500.0 | 83.33 |
| Croscarmellose sodium (Ac-Di-Sol) | 50.0 | 8.33 |
| Corn starch, plain (as 10% starch paste) | 17.0 | 2.83 |
| Microcrystalline cellulose (MCC) | 13.5 | 2.25 |
| Colloidal silicon dioxide | 13.5 | 2.25 |
| Magnesium stearate | 6.0 | 1.0 |
| **Total** | **600.0** | **100.0** |
| **Coat -** | | |
| Ethyl cellulose | 42.0 | coated to a weight gain of 9.8% by weight of the core. |
| Hydroxypropyl methylcellulose | 16.8 | |

The timed pulse release tablets were prepared as per the method given in Example 1 above. The timed pulse release tablets were subjected to dissolution studies using pH 6.8 buffer at 37+0.5°C, in a USP Type II apparatus (rpm = 75). The release profile for metformin is recorded in Table 5 below.

**Table 5**

| **Time (min)** | **% metformin released** |
|---|---|
| 45 | 1 |
| 120 | 91 ± 5.33 |

The tablets were found to release the metformin as a pulse after the rupture of the coat at a predetermined time. The tablets were tested in water, using different conditions of pH and apparatus, and the opening time is recorded in Table 6 below.

**Table 6**

| **No.** | **Medium** | **Conditions used** | **Opening time of 6 tablets (hours.min)** |
|---|---|---|---|
| 1. | pH 6.8 | USP Type I dissolution apparatus, with rpm of 100 | 1.15, 1.04, 1.16, 1.13, 1.21, 1.16 |
| 2. | pH 6.8 | USP Type I dissolution apparatus, with rpm of 100 | 1.37, 1.18, 1.20, 1.12,1.00, 1.15 |
| 3. | pH 6.8 | USP Type I dissolution apparatus, with rpm of 100 | 1.02, 1.15, 1.07, 1.10, 1.15, 0.53 |
| 4. | 0.1N HCl | USP Type II dissolution apparatus, with rpm of 75 | 1.11, 1.10, 0.50, 0.58, 0.59, 0.45 |
| 5. | pH 6.8 | USP Type II dissolution apparatus, with rpm of 50 | 1.00, 1.09, 0.55, 1.09,1.09, 1.22 |
| 6. | 0.1N HCl | USP Type I dissolution apparatus, with rpm of 100 | 1.02, 1.00, 1.23, 1.23, 1.26, 1.01 |

### Example 3

The timed pulse release composition of the present invention was subjected to radiological studies to determine the coat rupture time in vivo. The composition of Example 2 with the addition of 25 mg barium sulfate in the core was used for the radiological studies. The delayed release metformin tablet cores containing barium sulfate were prepared as per the method given in Example 1 with the barium sulfate being mixed with the starch paste to ensure its uniform distribution in the core.

A single dose, open label study was carried out using six healthy male volunteers. The subjects were fasted overnight before dosing and for 4 hours thereafter. Drinking water was prohibited for 2 hours before dosing and 2 hours thereafter. A single barium sulfate containing delayed release metformin tablet core was administered to each volunteer as the test product along with 240ml of drinking water. Standard meals were provided at 4 hours after dosing. X-rays were taken at the following time points after dosing - 30, 45, 60, 75 and 90 minutes. The result of the radiological follow-up at the above-mentioned time intervals is given in Table 7 below.

**Table 7**

| **Vol. No.** | **Position of the tablet (minutes)** | | | | |
|---|---|---|---|---|---|
| | **30** | **45** | **60** | **75** | **90** |
| 1 | Proximal small bowel (intact) | Proximal small bowel (intact) | Obscure (intact) | Left hyponchondrion of colon (intact) | Disappeared completely |
| 2 | Not observed | Not observed | Not observed | Not observed | Not observed |
| 3 | Small bowel (intact) | Small bowel (intact) | Obscure | Small bowel (intact) | Disappeared completely |
| 4 | Stomach fundus (intact) | Pyloric antrum (intact) | Pyloric antrum (intact) | Pyloric antrum (intact) | Proximal jejunal loop (Disintegrating) |
| 5 | Distal jejunal loop (intact) | Proximal ileal loop (intact) | Ileal loop (Disintegrating) | Ileal loop (Disintegrating) | Disappeared completely |
| 6 | Pyloric antrum (intact) | Pyloric antrum (intact) | Duodenojejunal flexure (intact) | Distal duodenum (disintegrating) | Disappeared completely |

As seen in Table 7 above, the tablet was not observed in volunteer no. 2, perhaps due to insufficient barium sulfate in the core. In four of the five remaining volunteers, the tablet was completely disintegrated in 90 minutes, and in volunteer no. 4 the tablet started disintegrating at 90 minutes. Thus, for the timed pulse release composition upon oral administration of the composition to human subjects, the coat ruptured in a reliable manner at about a predetermined time after oral administration of the composition.

### Example 4

The timed pulse release composition of the present invention was prepared as per the formula in Table 8 below.

**Table 8**

| **Ingredients** | **Quantity (mg)** | **Percent (%) w/w.** |
|---|---|---|
| **Intragranular** | | |
| Oxybutynin chloride | 3.3 | 3.66 |
| Microcrystalline cellulose (Avicel pH 101) | 50.0 | 55.56 |
| Lactose monohydrate | 18.2 | 20.22 |
| Crocarmellose sodium (Ac-Di-Sol) | 9.0 | 10.0 |
| Maize starch | 5.0 | 5.56 |
| **Extragranular** | | |
| Microcrystalline cellulose (Avicel pH 102) | 2.0 | 2.22 |
| Colloidal silica (Aerosil 200) | 2.0 | 2.22 |
| Magnesium stearate | 0.5 | 0.56 |
| **Total** | **90** | **100.0** |

Core tablets were prepared as described in Example 1. The cores were coated using the coating composition given in Table 9 below.

**Table 9**

| **Ingredients** | **% w/w** |
|---|---|
| Ethyl cellulose (Standard 20) | 3.75 |
| Hydroxypropyl methylcellulose (HPMC 50) | 1.25 |
| Dichloromethane | 76 |
| Methanol | 19 |

A coat rupture time of 4 hours could be obtained when the tablets were coated to a weight gain of 13-14%; and a coat rupture time of 8 hours could be obtained when the tablets were coated to a weight gain of 20%.

### Example 5

The timed pulse release composition of the present invention was prepared as per the formula in Table 10 below.

**Table 10**

| **Ingredients** | **Quantity (mg)** | **Percent (%) w/w of the core** |
|---|---|---|
| **Intragranular** | | |
| Carvedilol | 5.00 | 7.14 |
| Lactose | 34.00 | 48.57 |
| Microcrystalline cellulose | 12.00 | 17.14 |
| Starch | 10.00 | 14.29 |
| Croscarmellose sodium | 1.50 | 2.14 |
| Red oxide of iron | 0.5 | 0.71 |
| Polyvinylpyrrolidone (PVP K-30) | 2.00 | 2.86 |
| **Extragranular** | | |
| Croscarmellose sodium | 2.00 | 2.86 |
| Talc | 2.50 | 3.57 |
| Magnesium stearate | 1.00 | 1.43 |
| Colloidal silicon dioxide | 0.50 | 0.71 |

Core tablets were prepared as described in Example 1. The cores were coated using the coating composition given in Table 11 below.

**Table 11**

| **Ingredients** | **% w/w of the core** |
|---|---|
| Ethyl cellulose (M7) | 7.86 |
| Hydroxypropyl methylcellulose 2910 (HPMC ES) | 2.0 |
| Triethyl citrate | 0.71 |
| Talc | 0.43 |

A coat rupture time of 4 hours could be obtained when the tablets were coated to a weight gain of 11%; and a coat rupture time of 7 hours could be obtained when the tablets were coated to a weight gain of 13%.

### Comparative example 1

This comparative example illustrates the need for optimization of the composition to obtain, at about the predetermined time, a reliable manner of coat rupture.

Tablet cores were prepared according to the composition given in Table 12. The target coat rupture time was 1 hour.

**Table 12**

| **Ingredients** | **Quantity (mg)** |
|---|---|
| Metformin hydrochloride | 500.0 |
| Croscarmellose sodium (Ac-Di-Sol) | 34.5 |
| PVP K-90F | 10.0 |
| Magnesium stearate | 5.5 |
| **Total** | **550.0** |

The above cores were coated with a combination of ethylcellulose and hydroxypropyl methylcellulose dissolved in methylene chloride : methanol (4:1) solvent system. The ratio of ethylcellulose to hydroxypropyl methylcellulose was varied to evaluate its effect on the coat rupture time. When the ratio was 9:2 and the gain in weight upon coating was 4% by weight of the core, the coat rupture time was about 2 hours. The coat rupture time could be decreased by decreasing the amount of coat applied. However, at a ethylcellulose to hydroxypropyl methylcellulose ratio of 9:2, the coat rupture time was sensitive to this factor and this could lead to coat rupture time changing with variations in amount of coat applied from batch to batch. It was found that by a small change from 4% to 3% weight gain upon coating, the coat rupture time decreased to 45-60 minutes. Increase in proportion of hydroxypropyl methylcellulose decreased the coat rupture time. Ratios of ethyl cellulose to hydroxypropyl methylcellulose in the range of 8:3 to 7:3 were evaluated and it was surprisingly found that at these ratios coat rupture time of about 1 hr was obtained and the coat rupture time was not sensitive to the amount of coat applied. However, the coat did not rupture in a reliable manner as is evident from the results on the dissolution test evaluation for coat rupture time given in Table 13 below. The test was conducted in a USP type II apparatus in pH 6.8 buffer at 50 rpm.

**Table 13**

| **% weight gain on application of coat of EC: HPMC ratio of 7.5:3** | **No. of tablets tested** | **Opening time (minutes)** |
|---|---|---|
| 9% | 18 | 60, 53, 60, > 135, 60, 58, 48, 50, >135, 50, 75, 55, 65, 64, 55, 55, 55, 48 |
| 11% | 18 | 90, 71, 78, 80, > 150, 79, 60, 66, 73, 60, 91,70, 76, 85, did not open, 76, 76, did not open |
| 14.6% | 6 | 66, 65, 78, 180, 86, 60 |

It was seen that on an average the coat rupture time meets the target rupture time of about 1 hr, however, the reliability of rupture was low in that for some tablets the coat rupture was unduly prolonged. The coat composition was then kept fixed and the core composition was optimized, for example, to compositions in Examples 1 and 2, to achieve coat rupture and drug release in a reliable manner.

### Comparative example 2

The following example is generated as per example 1 of European patent EP 0408496. The tablets were made as per the formula in Table 14 below-

**Table 14**

| **Ingredients** | **Quantity (mg/tablet)** |
|---|---|
| **Core** | |
| Diclofenac sodium | 50mg |
| Polyvinylpyrrolidone (crosslinked) | 100mg |
| Sodium chloride | 50mg |
| Silica aerogel (Aerosil)^{®} 200) | 7mg |
| | |
| Magnesium stearate | 3mg |
| **Coating** | |
| Cellulose acetate (containing 32% acetyl) | 31mg |
| Cellulose acetate (containing 32.9% acetyl) | 32.33mg |
| Hydroxypropyl methylcellulose | 3.33mg |

The core components were mixed in a tumbler mixer and compressed in a tabletting press using a 8mm concave punch. The coating components were dissolved in a mixture of methylene chloride and methanol. This solution was used to coat the cores by a fluidized bed method. Three different batches were obtained by coating the cores to a weight gain of 4% and 9.8% (by weight of the core). The tablets were then dried for 48 hours.

The tablets obtained by this formula were tested in 900ml of water at 37°C and the opening time is recorded in Table 15 below.

**Table 15**

| **Coating (% by weight of the core)** | **Observations** | **Target opening time as per Table 1 of example of IE 902533** |
|---|---|---|
| 4% (before drying) | One tablet opened at about 45 minutes. Remaining tablets did not open till 3 hours and 20 minutes. | 65 minutes |
| 4% (after drying for 48 hours at 40°C) | One tablet opened at about 30 minutes, and another opened at about 50 minutes. Remaining tablets did not open till 2 hours and 15 minutes. | 65 minutes |
| 9.8% | No tablet opened till 2 hours and 56 minutes. | 120 minutes |

The above observations indicate that the tablets obtained by the formula mentioned in IE 902533 do not provide opening of the tablets at a specific predetermined time, as claimed in the main claim of the patent, in a reliable manner.

While the invention has been described with reference to specific embodiments, this was done for purposes of illustration only and should not be considered to limit the scope of the invention.

## Claims

1. A timed pulse release composition comprising:
a. a core composition comprising a therapeutically active agent, a swelling agent selected from the group consisting of cross-linked sodium carboxymethyl cellulose, crosslinked polyvinylpyrrolidone, and sodium starch glycolate, and optionally water soluble compound(s) for inducing osmosis, and
b. a coat composition comprising one or more film forming polymers, wherein the film forming polymers in the coat comprise a mixture of a water insoluble polymer and a water soluble polymer, wherein the ratio of water insoluble polymer to water soluble polymer ranges from 6:3 to 9:3; wherein the water insoluble polymer is ethyl cellulose and the water soluble polymer is hydroxypropyl methyl cellulose;
wherein the swelling agent swells considerably but does not form a strong gel and is present in sufficient quantity in the core to provide upon uptake of water a swelling pressure in excess of cohesive strength of the coating surround the core, such that upon imbibing fluid from the surrounding the core swells, and the coat ruptures to release in a pulse, the therapeutically active agent in a reliable manner at about a predetermined time, wherein the predetermined time is in the range of 1 to 12 hours, wherein the reliable manner of rupture comprises rupturing of 36 tablets out of a total of 36 tablets at the predetermined time when tested by subjecting the tablets to USP dissolution test using an aqueous media at 37±0.5°C, in a USP Type I or Type II apparatus at an rpm selected from the range of 50 rpm to 100 rpm.

2. A timed pulse release composition as claimed in claim 1 wherein upon oral administration of the composition to human subjects, the coat ruptures in a reliable manner at a predetermined time after oral administration of the composition.

3. A timed pulse release composition as claimed in claim 1 wherein the predetermined time is in the range from 1 hour to 4 hours, and the 36 out of the 36 tablets rupture within ± 50% of the predetermined time.

4. A timed pulse release composition as claimed in claim 1 wherein the predetermined time is in the range from > 4 hours to 12 hours, and the 36 out of 36 tablets rupture within ± 25% of the predetermined time.

5. A timed pulse release composition as claimed in claim 4, wherein the core further comprises a wicking agent.

6. A timed pulse release composition as claimed in claim 5 wherein the wicking agent is selected from microcrystalline cellulose and colloidal silicon dioxide.

7. A timed pulse release composition as claimed in claim 6, wherein the core further comprises starch.

## Patentansprüche

1. Zusammensetzung mit zeitlich getakteter Freisetzung, umfassend:
a. eine Kernzusammensetzung, die ein therapeutisch aktives Mittel, ein aus der aus vernetzter Natriumcarboxymethylcellulose, vernetztem Polyvinylpyrrolidon und Natriumstärkeglykolat bestehenden Gruppe ausgewähltes Quellmittel und gegebenenfalls (eine) wasserlösliche Verbindung(en) zum Induzieren von Osmose umfasst, und
b. eine Hüllzusammensetzung, die ein oder mehrere filmbildende Polymere umfasst, worin die filmbildenden Polymere in der Hülle ein Gemisch aus einem wasserunlöslichen Polymer und einem wasserlöslichen Polymer umfassen, worin das Verhältnis zwischen wasserunlöslichem Polymer und wasserlöslichem Polymer von 6:3 bis 9:3 reicht, worin das wasserunlösliche Polymer Ethylcellulose ist und das wasserlösliche Polymer Hydroxypropylmethylcellulose ist;
worin das Quellmittel beträchtlich anschwillt, aber kein starkes Gel bildet und in hinlänglicher Menge im Kern vorliegt, um bei Aufnahme von Wasser einen die Kohäsionsfestigkeit der Beschichtung rund um den Kern übersteigenden Quellungsdruck bereitzustellen, so dass bei Aufsaugen von Wasser aus der Umgebung der Kern anschwillt und die Hülle aufbricht, um das therapeutisch aktive Mittel in einem Takt verlässlich etwa zu einer festgesetzten Zeit freizusetzen, worin die festgesetzte Zeit im Bereich von 1 bis 12 h liegt, worin die Verlässlichkeit des Bruches das Aufbrechen von 36 Tabletten von 36 Tabletten in der festgesetzten Zeit umfasst, wenn eine Testung durch Aussetzen der Tabletten gegenüber dem USP-Auflösungstest unter Verwendung eines wässrigen Mediums bei 37 ± 0,5 °C in einer USP-Vorrichtung des Typs I oder des Typs II mit einem aus dem Bereich von 50 U/min bis 100 U/min ausgewählten U/min-Wert erfolgt.

2. Zusammensetzung mit zeitlich getakteter Freisetzung nach Anspruch 1, worin bei oraler Verabreichung der Zusammensetzung an menschliche Individuen die Hülle verlässlich zu einer festgesetzten Zeit nach der oralen Verabreichung der Zusammensetzung aufbricht.

3. Zusammensetzung mit zeitlich getakteter Freisetzung nach Anspruch 1, worin die festgesetzte Zeit im Bereich von 1 bis 4 h liegt und das Aufbrechen von 36 der 36 Tabletten in ± 50 % der festgesetzten Zeit erfolgt.

4. Zusammensetzung mit zeitlich getakteter Freisetzung nach Anspruch 1, worin die festgesetzte Zeit im Bereich von > 4 bis 12 h liegt und das Aufbrechen von 36 der 36 Tabletten in ± 25 % der festgesetzten Zeit erfolgt.

5. Zusammensetzung mit zeitlich getakteter Freisetzung nach Anspruch 4, worin der Kern weiters ein feuchtigkeitsanziehendes Mittel umfasst.

6. Zusammensetzung mit zeitlich getakteter Freisetzung nach Anspruch 5, worin das feuchtigkeitsanziehende Mittel aus mikrokristalliner Cellulose und kolloidem Siliciumdioxid ausgewählt ist.

7. Zusammensetzung mit zeitlich getakteter Freisetzung nach Anspruch 6, worin der Kern weiters Stärke umfasst.

## Revendications

1. Composition à libération par impulsion réglée dans le temps comprenant:
a. une composition de base comprenant un agent thérapeutiquement actif, un agent gonflant sélectionné dans le groupe consistant en sodium carboxyméthyle cellulose réticulé, polyvinylpyrrolidone réticulé et glycolate d'amidon sodique et, en option un ou des composés solubles dans l'eau pour induire l'osmose, et
b. une composition de revêtement comprenant un ou plusieurs polymères de formation de film, où les polymères de formation de film dans le revêtement comprennent un mélange d'un polymère insoluble dans l'eau et d'un polymère soluble dans l'eau, où le rapport du polymère insoluble dans l'eau au polymère soluble dans l'eau est de 6:3 à 9:3, où le polymère insoluble dans l'eau est l'éthyl cellulose, et le polymère soluble dans l'eau est l'hydroxypropyl méthyl cellulose;
où l'agent de gonflage gonfle considérablement mais ne forme pas un gel fort et est présent en une quantité suffisante dans la base pour réaliser, lors de l'absorption d'eau, une pression de gonflage supérieure à la force de cohésion du revêtement entourant la base de sorte que lorsqu'elle est imbibée de fluide de l'environnement, la base gonfle, et le revêtement se rompt pour libérer par une impulsion l'agent thérapeutiquement actif d'une manière fiable environ à un temps prédéterminé, où le temps prédéterminé est dans la plage de 1 à 12 heures, où la manière fiable de la rupture comprend la rupture de 36 tablettes d'un total de 36 tablettes au temps prédéterminé lors d'un test, en soumettant les tablettes à un test de dissolution USP en utilisant un milieu aqueux à 37 ± 0,5°C, dans un appareil de Type I ou de Type II de USP à une vitesse de rotation sélectionnée dans la plage de 50 tr/min à 100 tr/min.

2. Composition à libération par impulsion réglée dans le temps selon la revendication 1, dans laquelle lors d'une administration orale de la composition à des sujets humains, le revêtement se rompt d'une manière fiable à un temps prédéterminé après l'administration orale de la composition.

3. Composition à libération par impulsion réglée dans le temps selon la revendication 1, dans laquelle le temps prédéterminé est dans la plage de 1 heure à 4 heures, et 36 parmi les 36 tablettes se rompent durant ± 50% du temps prédéterminé.

4. Composition à libération par impulsion réglée dans le temps selon la revendication 1, dans laquelle le temps prédéterminé est dans la plage de > 4 heures à 12 heures, et les 36 parmi les 36 tablettes se rompent durant ± 25% du temps prédéterminé.

5. Composition à libération par impulsion réglée dans le temps selon la revendication 4, où la base comprend en outre un agent à effet de mèche.

6. Composition à libération par impulsion réglée dans le temps selon la revendication 5, dans laquelle l'agent à effet de mèche est sélectionné parmi la cellulose microcristalline et le dioxyde de silicium colloïdal.

7. Composition à libération par impulsion réglée dans le temps selon la revendication 6, dans laquelle la base comprend en outre de l'amidon.
